# EUROPEAN PATENT SPECIFICATION

(11) **EP 4 243 692 B1**
(45) Date of publication and mention of the grant of the patent: **01.07.2026**
(21) Application number: 21811490.8
(22) Date of filing: 10.11.2021
(51) Int. Cl.: A61B 5/16, A61B 5/00, G16H 10/20, A61B 5/11

(54) **SYSTEM AND METHOD FOR CLASSIFYING AND USING CHRONOTYPES**
SYSTEM UND VERFAHREN ZUR KLASSIFIZIERUNG UND VERWENDUNG VON CHRONOTYPEN
SYSTÈME ET PROCÉDÉ DE CLASSIFICATION ET D'UTILISATION DE CHRONOTYPES

(30) Priority: 10.11.2020 US 202063112017 P; 29.01.2021 US 202163143386 P
(43) Date of publication of application: 20.09.2023
(73) Proprietor: ResMed Pty Ltd, Bella Vista, New South Wales 2153 (AU)
(72) Inventor: SHAW, Matthew, James, Bella Vista, New South Wales 2153 (AU); ARIDI, David, Zeid, Bella Vista, New South Wales 2153 (AU); MEALEY, Nicholas, James, Bella Vista, New South Wales 2153 (AU); XU, Jessica, Dublin, D18 T6T7 (IE); VITHANAGE FERNANDO, Varuni Lakshana, Bella Vista, New South Wales 2153 (AU)
(74) Representative: Mathys & Squire
(86) International application number: PCT/IB2021/060425
(87) International publication number: WO 2022/101810

(56) References cited:
- WO-A1-2020/002763
- US-A1- 2014 323 828
- US-A1- 2016 270 718
- US-A1- 2017 238 868
- US-A1- 2020 077 942
- US-B1- 10 546 108

## Description

### PRIORITY CLAIM

The application claims priority to and the benefit of U.S. Provisional Patent Application No. 63/112,017 filed on November 10, 2020 and U.S. Provisional Patent Application No. 63/143,386 filed on January 29, 2021.

### TECHNICAL FIELD

The present disclosure relates generally to health monitoring systems, and more specifically to determining a chronotype classification for a user and display of information based on the determined chronotype classification.

### BACKGROUND

Humans have an internal body clock that sets optimal sleep and wake times. Body clocks vary by individual and, as is widely known, there may be morning persons and evening persons based on the time of day the individual feels most alert. Energy refers to the user's baseline store of energy for the day. Energy is based on how well the user slept, and thus a poor night's sleep results in low energy for the entire ensuing day). Alertness refers to the user's mental and physical capacity at a particular time of day. Alertness fluctuates based on the body clock of an individual. The energy level for the whole day is determined at the beginning of the day (based on the previous night's sleep) and does not change throughout the day. The user can have high, moderate, or low energy for the day. Alertness, however, changes throughout the day and is classified as peak, falling, base, and rising alertness.

An individual body clock may be defined by the chronotype of the individual. For example, there may be three chronotypes that may be used to classify individuals: morning, day, and night. A morning chronotype (an extreme Lark) experiences natural awakening, and is alert early in the day. The morning chronotype is ready for bed in the early evening. A day chronotype (Lark) awakens later, and their peak alertness is later in the day. A night chronotype (an Owl) rises as late as possible and maintains alertness into the night.

Knowing the patterns of energy of a specific chronotype classification can help give an understanding of when an individual prefers to fall asleep. This assists sleep disorder diagnosis. The connection between sleep and wake time may be defined by the specific chronotype (connecting a night period to a day period relative to the chronotype). In addition, classification of the chronotype allows an individual to determine when one is more likely to be alert and how to get the most energy out of a day.

Further, a chronotype classification dictates the times during a day when an individual is most productive for corresponding activities. The chronotype classification also determines ideal times for eating, particularly when aiming to both stay energized and make sure sleep is not disrupted. Unfortunately, there is currently no accessible routine that allows a user to determine their classification chronotype. Further, even if an individual understands their chronotype, they do not have sufficient information to schedule activities to best match alertness levels as defined by their chronotype.

There is therefore a need for a system that allows a user to determine their chronotype. There is a further need for a system that suggests times based on chronotype to maximize energy for different activities. There is also a need for a wearable device that provides notifications at different times based on a determined chronotype.

WO 2020/002763 A1 discloses system a system for guiding a user to improve the general performance of sleep, physical action and/or cognitive action based on analysis and processing of data related to cardiac rhythm of a user.

US 2017/238868 A1 discloses a wearable device for monitoring and predicting alertness of an individual.

US 2020/077942 A1 discloses systems, methods, and articles for stress reduction and sleep promotion. A stress reduction and sleep promotion system includes at least one remote device, at least one body sensor, and at least one remote server.

### SUMMARY

The invention relates to a method to display alertness to a user. A chronotype classification is determined for the user. An alertness waveform associated with alertness levels at different times is aligned based on the chronotype classification. The alertness levels from the alertness waveform are displayed to the user on a display of a wearable device. An alertness test is displayed on the wearable device. The result of the alertness test from the user are received and correlated with an alertness level determined from the alertness waveform. An average alertness score is determined based on the result of a series of alertness tests taken at either a peak point or a base point of the alertness waveform.

In other implementations of the disclosed example method, the method also includes displaying notifications for optimal activities at a predetermined time based on the chronotype classification. In another implementation, the notifications include icons corresponding to one of the optimal activities displayed on the display. In another implementation, the notifications include icons corresponding to one of the optimal activities displayed on the display. In another implementation, the method includes displaying a schedule interface displaying a schedule of activities for the user that includes an input to allow the user insert an optimal activity into the schedule of activities. In another implementation, the method includes displaying a notification for an optimal sleep time based on the chronotype classification. In another implementation, the chronotype is determined based on the user answering questions from a questionnaire. In another implementation, the chronotype is determined based on physiological data measured from the user. In another implementation, the physiological data is determined by a physiological sensor on the wearable device. In another implementation, the physiological data is analyzed to determine sleep and wake times, and activity levels through a day to determine the chronotype. In another implementation, operational data from a therapy device is analyzed to determine sleep and wake times, and activity levels through a day to determine the chronotype. In another implementation, the determination is performed with a machine learning model trained with data collected from a user population and the chronotype of each of the user population. In another implementation, the method includes displaying notifications to eat or take stimulants based on the chronotype. In another implementation, the method includes displaying notifications to exercise or work based on the chronotype. In another implementation, the method includes measuring sleep related data from the user and displaying a predicted energy level based on the measured sleep related data. In another implementation, the sleep related data is a sleep score for the user. In another implementation, the method includes displaying a visual indicator on the wearable device that enables the display of a website on a web-enabled device scanning the visual indicator. In another implementation, the visual indicator is a QR code. In another implementation, the website is one of a virtual coach website, an information website, or an instruction website. In another implementation, the wearable device includes a transceiver that allows communication with a mobile device. In another implementation, a graphical representation displayed on the display of the wearable device changes based on the time period and the alertness waveform. In another implementation, the method includes collecting additional data relating to the chronotype of the user subsequent to the chronotype classification. The method also includes adjusting the alertness waveform based on the collected data. In another implementation, the method includes alerting a user of changes in alertness level based on the alertness waveform. In another implementation, the method includes displaying an alertness test on the wearable device; receiving a result of the alertness test from the user; and correlating the result of the alertness test with an alertness level determined from the alertness waveform. In another implementation, the alertness test is one of a short-term memory test, a reaction test, or a Stroop test. In another implementation, the method includes determining an average alertness score based on the result of a series of alertness tests taken at either a peak point or a base point of the alertness waveform. In another implementation, the method includes adjusting the alertness waveform based on the result of the alertness test. In another implementation, the wearable device is a wrist mounted. In another implementation, the alertness levels are displayed in correlation to predetermined colors on the display.

The invention further relates to computer program product comprising instructions which, when executed by a computer, cause the computer to carry out the above described method. The computer program product can be a non-transitory computer readable medium.

The invention further relates to a wearable device allowing the display of alertness for a user. The wearable device includes a display and a controller coupled to the display. The controller is operable to read an input of a chronotype classification of the user. The controller aligns an alertness waveform relating to alertness of the user relative to time periods based on the chronotype classification. The controller displays the alertness waveform and an alertness test on the display. The result of the alertness test is received from the user and correlated with an alertness level determined from the alertness waveform. An average alertness score is determined based on the result of a series of alertness tests taken at either a peak or base points of the alertness waveform.

In other implementations of the disclosed example wearable device, the controller displays notifications for optimal activities on the display at a predetermined time based on the chronotype classification. In another implementation, the notifications include icons corresponding to one of the optimal activities displayed on the display. In another implementation, the controller generates a schedule interface on the display displaying a schedule of activities for the user that includes an input to allow the user insert an optimal activity into the schedule of activities. In another implementation, the display displays a notification for an optimal sleep time based on the chronotype classification. In another implementation, the chronotype is determined based on the user answering questions from a questionnaire. In another implementation, the chronotype is determined based on physiological data measured from the user. In another implementation, the wearable device includes a physiological sensor that measures the physiological data. In another implementation, the physiological data is analyzed to determine sleep and wake times, and activity levels through a day to determine the chronotype. In another implementation, operational data from a therapy device is analyzed to determine sleep and wake times, and activity levels through a day to determine the chronotype. In another implementation, the determination is performed with a machine learning model trained with data collected from a user population and the chronotype of each of the user population. In another implementation, the controller is operable to display notifications to eat or take stimulants based on the chronotype on the display. In another implementation, the controller is operable to display notifications to exercise or work based on the chronotype on the display. In another implementation, the controller is operable to receive measured sleep related data from the user and display a predicted energy level based on the measured sleep related data on the display. In another implementation, the sleep related data is a sleep score for the user. In another implementation, the controller is operable to display a visual indicator on the display that enables the display of a website on a web-enabled device scanning the visual indicator. In another implementation, the visual indicator is a QR code. In another implementation, the website is one of a virtual coach website, an information website, or an instruction website. In another implementation, the wearable device includes a transceiver that allows communication with a mobile device. In another implementation, the display changes a graphical representation based on the time period and the alertness waveform. In another implementation, the controller is operable to collect additional data relating to the chronotype of the user subsequent to the chronotype classification, and adjust the alertness waveform based on the collected data. In another implementation, the controller is operable to alert a user of changes in alertness level based on the alertness waveform. In another implementation, the controller is operable to: display an alertness test on the display; receive a result of the alertness test from the user; and correlate the result of the alertness test with an alertness level determined from the alertness waveform. In another implementation, the alertness test is one of a short-term memory test, a reaction test, or a Stroop test. In another implementation, the controller is operable to determine an average alertness score based on the result of a series of alertness tests taken at either a peak or base points of the alertness waveform. In another implementation, the controller is operable to adjust the alertness waveform based on the result of the alertness test. In another implementation, the wearable device is a wrist mounted. In another implementation, the alertness levels are displayed in correlation to predetermined colors on the display.

Also disclosed herein is a system to classify a user by a chronotype. The system includes a server operable to display a web-interface having a questionnaire for classification of a chronotype. A web-enabled device has a network interface sending inputs to the questionnaire to determine the classification of the chronotype based on the inputs and displaying the resulting chronotype to a user. A database stores information relating to the user and the chronotype classification. A wearable device is operated by the user executing an application to display a waveform showing alertness. The application aligns the waveform to time periods based on the determined chronotype.

The invention is defined by the appended claims.

### BRIEF DESCRIPTION OF THE DRAWINGS

The disclosure will be better understood from the following description of exemplary embodiments together with reference to the accompanying drawings, in which:
FIG. 1 shows a system that provides users notifications and information in relation to their determined chronotype;
FIG. 2A is a diagram of the components of a computing device in the system of FIG. 1;
FIG. 2B is a block diagram of the components of a wearable device used to determine a chronotype;
FIG. 3 is a flow diagram of the process of on-boarding a wearable device to determine a chronotype;
FIGs. 4A-4D shows screen images of interfaces on an example wearable device in FIG. 1 for determining chronotypes and scheduling activities based on the chronotype;
FIGs. 5A-5E are screen images of interfaces on an example wearable device that display an alertness sinusoid, schedule, energy, and sleep data based on the alertness sinusoid;
FIGs. 6A-6G are screen images of interfaces on an example wearable device that provide tests, tips, and assistance to a user;
FIGs. 7A-7B is a state diagram of the features of the example wearable application that are operated by the wearable device in FIG. 1;
FIG. 8 is a chart of questions to determine the chronotype of an individual;
FIG. 9 is a chart showing different times of alertness for different chronotypes;
FIG. 10 is an example of tips that may be generated for the user at different times according to the chronotype;
FIG. 11 is a table of notifications that are available based on the chronotype that may be activated by the wearable device;
FIG. 12 is a block diagram showing the interactions between the wearable device and application and a mobile device; and
FIG. 13 is a screen image of an example settings interface for the wearable application.

The present disclosure is susceptible to various modifications and alternative forms. Some representative embodiments have been shown by way of example in the drawings and will be described in detail herein. It should be understood, however, that the invention is not intended to be limited to the particular forms disclosed.

### DETAILED DESCRIPTION OF THE ILLUSTRATED EMBODIMENTS

The present inventions can be embodied in many different forms. Representative embodiments are shown in the drawings, and will herein be described in detail. The present disclosure is an example or illustration of the principles of the present disclosure, and is not intended to limit the broad aspects of the disclosure to the embodiments illustrated. To that extent, elements and limitations that are disclosed, for example, in the Abstract, Summary, and Detailed Description sections, but not explicitly set forth in the claims, should not be incorporated into the claims, singly or collectively, by implication, inference, or otherwise. For purposes of the present detailed description, unless specifically disclaimed, the singular includes the plural and vice versa; and the word "including" means "including without limitation." Moreover, words of approximation, such as "about," "almost," "substantially," "approximately," and the like, can be used herein to mean "at," "near," or "nearly at," or "within 3-5% of," or "within acceptable manufacturing tolerances," or any logical combination thereof, for example.

The present disclosure is a system that allows categorization of users (such as patients) into different "chronotypes" based on their body clock, i.e., are you a morning person or a night owl. A series of survey questions are used to determine a chronotype classification for an individual. The disclosure also includes a wearable device with a display that represents a body clock based on the determined chronotype for an individual.

The wearable device generates interfaces that make users aware of their chronotype and provides suggestions to maximize the energy for a day based on the chronotype. The wearable device may execute an application to determine a chronotype for a user and display a representation of a body clock to the user. The body clock is tailored to the determined chronotype, and causes indications to be communicated to the user or displayed with the body clock at different times of the day. The body clock may be personalized further based on other sensor data determined by the wearable device or other devices in communication with the wearable device. The information may allow a user to receive notifications and tips on how best to maximize their energy during certain times of the day that are associated with their chronotype classification. This may also assist in providing better sleep based on the collected data and chronotype classification of the user.

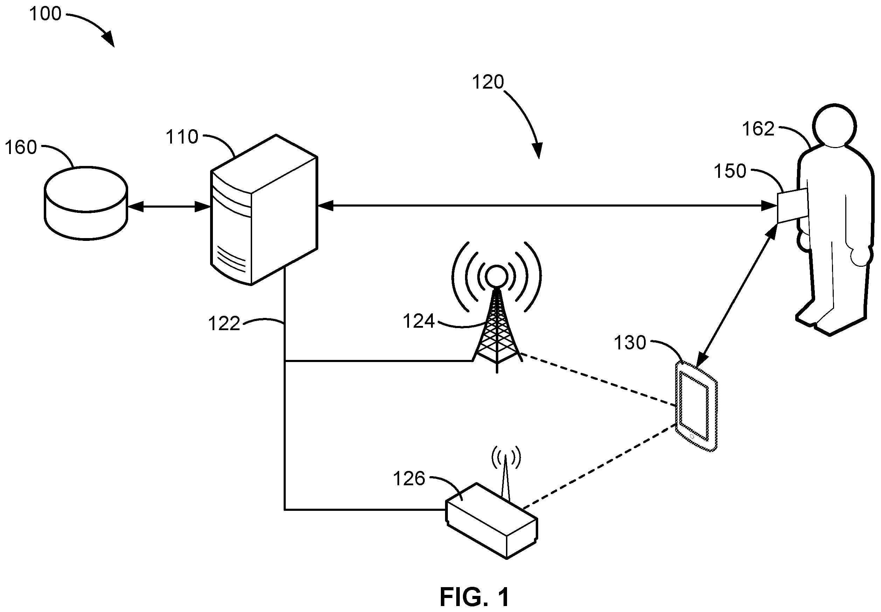

FIG. 1 depicts an example data collection system 100 that may be implemented for determining a chronotype, and then providing information tailored to a chronotype to assist users with functions such as efficient scheduling, maximizing energy and avoiding potential sleep disorders. The data collection system 100 may generally include one or more of servers 110, a communication network 120, and a mobile computing device 130. The server 110 and mobile computing device 130 may communicate via communication network 120, which may be a wired network 122, wireless network 124, or wired network with a wireless link 126. In some versions, the server 110 may communicate one-way with mobile computing device 130 by providing information to mobile computing device 130, or vice versa. In other embodiments, server 110 and computing device 130 may share information and/or processing tasks. The system may be implemented, for example, to permit integration of chronotype and other information relating to the users as described in more detail herein.

A database 160 is provided to collect data on a user population represented by a user 162. The database 160 may include additional relevant collected data relating to the user that may be accessed by the server 110 for other analysis such as providing information for activities, avoiding sleep disorders, and nutrition. In this example, the database 160 will include a determined chronotype for each user in the user population.

A user 162 may have access to the mobile computing device 130, which may be a mobile phone or a tablet. In this example, the user 162 may wear a wearable device 150 that may have computing functions including communication with the network 120. In this example, the wearable device is a wrist mounted device such as an Apple watch or a Fitbit. The wearable device 150 may be paired with the mobile device 130 through a relatively short range communication protocol such as Bluetooth. As will be explained below, the wearable device 150 may also include a physiological sensor that collects physiological data. In this example, the sensor may include heart rate sensors, oxygen level sensors, ECG sensors, pulse rate sensors, and the like. The sensors on the wearable device 150 may communicate with the mobile device 130 to record physiological data of the user 162. Alternatively, the displays and interfaces of the wearable device may be generated on the display of the mobile device 130. Some or all of the functions of the wearable device may also be executed by the mobile device 130.

The wearable device 150 may include a display and an interface that allows the determination of a chronotype of the user. In this example, the wearable device 150 has a processor that allows the execution of various wearable applications including an application for issuing tips, notifications, and information relating to an individual's alertness levels during the day according to their chronotype.

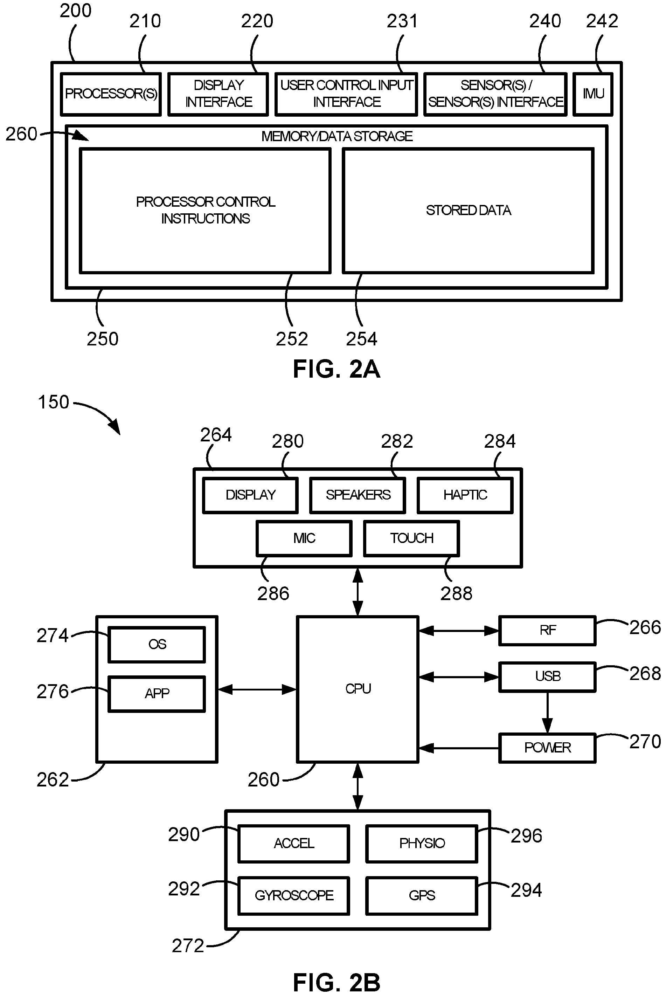

The mobile computing device 130 can be a mobile device, such as the smartphone or the tablet. Alternatively, the functions of the mobile computing device 130 may be performed by a desktop or laptop computer. FIG. 2A depicts the general architecture 200 of the computing device 130. The computing device 130 may include one or more processors 210. The computing device 130 may also include a display interface 220, a user control/input interface 231, a sensor 240 and/or a sensor interface for one or more sensor(s), an inertial measurement unit (IMU) 242 and a non-volatile memory/data storage 250.

Sensor 240 may be one or more cameras (e.g., a CCD charge-coupled device or active pixel sensors) that are integrated into computing device 130, such as those provided in a smartphone or in a laptop. Alternatively, where computing device 130 is a desktop computer, computing device 130 may include a sensor interface for coupling with an external camera, such as a webcam.

User control/input interface 231 allows the user to provide commands or respond to prompts or instructions provided to the user. This could be a touch panel, keyboard, mouse, microphone, and/or speaker, for example.

Display interface 220 may include a monitor, LCD panel, or the like to display prompts, output information (such as facial measurements or interface size recommendations), and other information, such as a capture display, as described in further detail below.

Memory/data storage 250 may be the computing device's internal memory, such as RAM, flash memory or ROM. In some embodiments, memory/data storage 250 may also be external memory linked to computing device 130, such as an SD card, server, USB flash drive or optical disc, for example. In other embodiments, memory/data storage 250 can be a combination of external and internal memory. Memory/data storage 250 includes stored data 254 and processor control instructions 252 that instruct processor 210 to perform certain tasks. Stored data 254 can include data received by sensor 240, such as a captured image, and other data that is provided as a component part of an application. Processor control instructions 252 can also be provided as a component part of an application.

FIG. 2B is a block diagram of the components of the wearable device 150 in FIG. 1. The wearable device 150 includes a processor (such as central processing unit (CPU) 260), a memory 262, a user interface 264, a radio frequency (RF) interface 266, a connector interface 268, a power source 270, and environmental sensors 272.

The memory 262 may be flash memory or other semiconductor memory (e.g., DRAM, SRAM). The memory 262 stores an operating system 274 executed by the processor 260 for operating the wearable device. The memory 262 stores data generated by wearable applications such as chronotype related data as will be explained below. The memory 262 also stores one or more wearable applications that may be executed by the processor 260 including a chronotype management application 276 (or wearable application 276).

The user interface 264 allows the user to provide inputs and receive outputs from the wearable device 150. The user interface 264 allows connection to a display 280, speakers 282, and a haptic actuator 284. The display 280 is a compact display such as an LCD (liquid crystal display), LED (light-emitting diode) display, or an OLED (organic light-emitting diode).

The user interface 264 also include various input devices such as a microphone 286 and a touch sensor 288. The touch sensor 288 may be a capacitive sensor array with the ability to localize contacts to a particular point or region on the surface of the sensor. In this example, the touch sensor 288 is overlaid over the display 280 to provide a touchscreen interface.

The processor 260 controls the operation of the wearable device 150 and executes the wearable programs stored in the memory 262. The processor 260 executes the operating system (OS) 274 and various applications for interfacing with a host device such as the mobile device 130 in FIG. 1.

The RF interface 266 can allow wearable device 150 to communicate wirelessly using different protocols with various host devices. The RF interface 266 may include RF transceiver components such as an antenna and supporting circuitry to enable data communication over a wireless medium, e.g., using Wi-Fi (IEEE 802.11 family standards), Bluetooth^{®} (a family of standards promulgated by Bluetooth SIG, Inc.), or other protocols for wireless data communication. In this example, the RF interface 266 provides near-field communication ("NFC") capability to support wireless data exchange between devices over a very short range.

The connector interface 268 allows the wearable device 150 to communicate with various host devices via a wired communication path. In this example, the connector interface 268 is a Universal Serial Bus (USB) port. The connector interface 268 is also a power port, allowing the wearable device 150 to receive power. The connector interface 268 may also provide connections for audio and/or video signals, which may be transmitted to or from a host device in analog and/or digital formats.

The power source 270 provides power for the wearable device 150. In this example, the power source 270 is a rechargeable battery and circuitry operable to charge the battery when the connector interface 268 is connected to a power source.

The environmental sensors 272 provide digital signals to the processor 260 on a streaming basis or in response to polling. The environmental sensors include an accelerometer 290, a gyroscope 292, and a GPS receiver 294. The environmental sensors 272 provide information about the location and/or motion of wearable device 150. Other sensors can also be included in addition to or instead of these examples. For example, a sound sensor can incorporate the microphone 286. Other sensors such as a physiological sensor 296 may provide physiological data from the wearer. The physiological sensor 296 may include sensors such as heart rate sensors, oxygen level sensors, ECG sensors, pulse rate sensors, or the like.

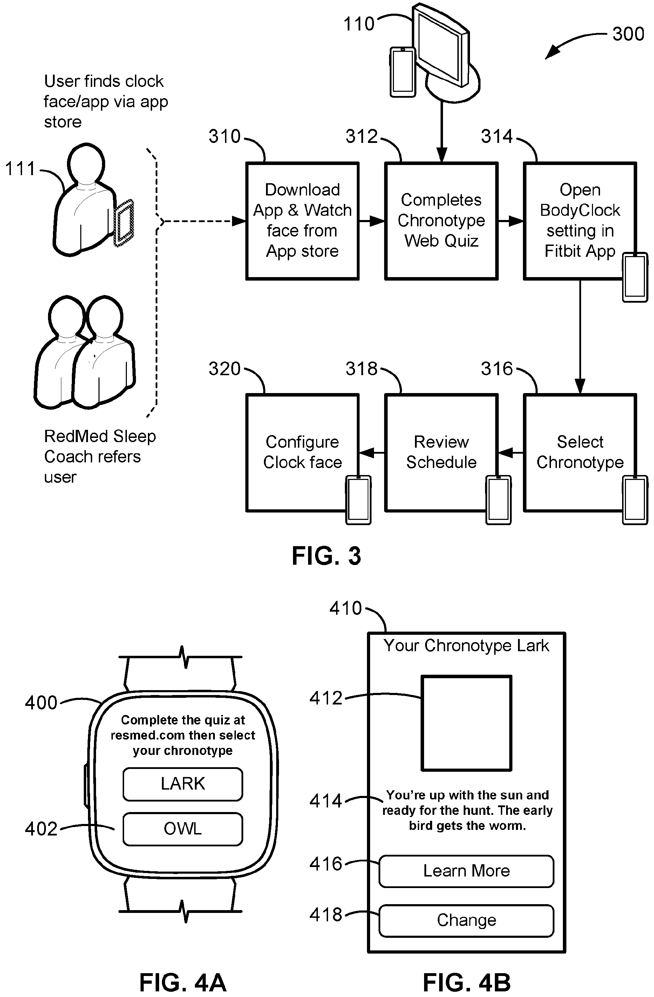

FIG. 3 is a flow diagram of the process of setting up the wearable device 150 in FIG. 2 to determine the chronotype of the user. The process also tailors the application 276 on the wearable device 150 to generate notifications and information based on a specific chronotype classified for the user. The wearable application 276 may be downloaded from an application store or other network source (310). The user may complete a chronotype web-based quiz on a computing device such as the mobile device 130 in FIG. 1 (312). The user opens the body clock settings in the wearable application 276 on the wearable device 150 (314). The user then selects the applicable chronotype (316) based on the quiz results that are displayed on the web interface. Alternatively, the results of the quiz may be sent automatically to the wearable device 150 and incorporated by the wearable application 276. The user may then review a schedule based on activities generated by the wearable application 276 (318). The user may configure the clock face interface of the wearable device 150 to select between different displays of the user specific energy curve as will be explained below (320). This may be controlled by the wearable application 276 or the operating system software of the wearable device.

FIGs. 4A-4D shows a series of interfaces that are generated by the application 276 running on the wearable device 150 in FIG. 2 that allow scheduling of activities, display of tips and information, and reminders, based on a determined chronotype. The interfaces are displayed on the display 280 of the example wearable device 150. In this example, the wearable device 150 may be wrist mounted such as an Apple Watch or a FitBit device.

A personalized day planner, such as a wearable body clock, can alert the user of the wearable device 150 when peak alertness times or lull times are, and suggest different activities based on the chronotype and time of day to maximize performance. These alerts may include when to start work, when to do analytical work (cognitively demanding activity), when to do administrative work (non-cognitively demanding activity), when to take stimulants such as caffeine, when to exercise, when to eat throughout the day, when to wind down, and when to sleep. The alerts may also include sleep hygiene tips throughout the day that can help improve sleep quality for the user based on the chronotype classification.

The wearable application 276 may be accessed through a generic interface generated by the operating system 274 of the wearable device 150 in FIG. 2. A survey response interface 400 shown in FIG. 4A directs a user to the website that allows the display of a series of interactive questions and answer fields that allow collection of user data for purposes of determining the chronotype of the user. The user data including the results of the quiz from the web interface is analyzed to determine the classification of a chronotype for the user. In this example, the website will output a chronotype for the user. The interface 400 displays a series of options 402 corresponding to each chronotype. In this example, the options 402 may include Lark, Extreme Lark, Owl, and Extreme Owl. Additional chronotypes may be displayed with corresponding tailored alertness curves. Alternatively, the website may automatically send the determined chronotype to the wearable device 150. The quiz may also be displayed on an interface generated by the application 276 on the wearable device 150.

FIG. 4B shows a result interface 410 that is displayed after the user answers the questions in the survey website and enters the chronotype to the interface 400 in FIG. 4A. The result interface 410 also includes a general description 412 of the entered chronotype. The result interface 410 also may include a detailed description 414 that includes information on optimal times for activities based on the chronotype. The result interface 410 may also include a learn more button 416 and a settings button 418. The learn more button 416 allows a user to access other sources of information such as external websites in relation to the entered chronotype. The settings button 418 allows a user to change the chronotype settings.

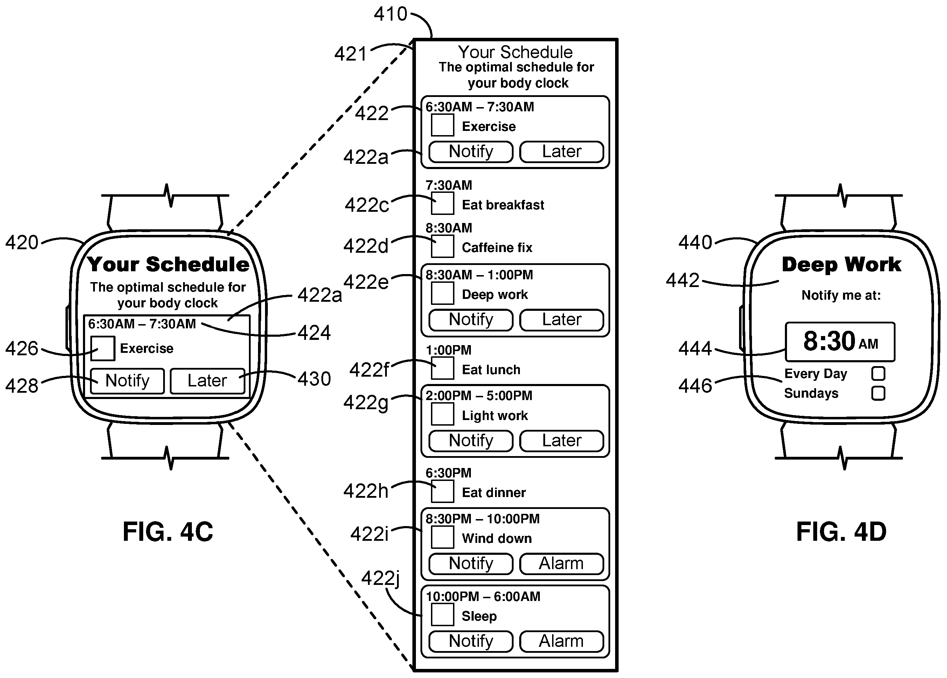

FIG. 4C shows a schedule interface 420 that displays an optimal schedule based on the chronotype of the user. The activities in the schedule includes certain default activities (deep work, light work, exercise, wind down, and sleep) as well optional activities (breakfast, caffeine, lunch, and dinner) that may be selected by the user. The entire schedule may be scrolled through on the interface 420 as shown by an expanded view 421. The schedule includes activity blocks 422 for activities that are matched to the timing of alertness level dictated by the chronotype of the user. In this example, activity blocks 422a-422j from an optimal schedule for the user may be displayed on the schedule interface 420. The activity blocks 422a-422j will be scheduled according to optimal efficiency from the chronotype that the user belongs to. In this example, the activity blocks 422a-422j are arranged chronologically. The example activity block 422a includes a time range 424, a description of the activity 426, a notify button 428, and a later button 430. The notify button 428 allows the user to set up an alert for the activity that activates an alert interface such as that shown in FIG. 4D when the time occurs to perform the activity. The later button 430 allows users to indicate that they could not or did not want to perform the activity at the proposed optimal time, in which case the activity may be moved later in the day to the second most optimal time. In this example the activities include eating breakfast, taking caffeine, deep work, eating lunch, light work, eating dinner, wind down, and sleep as represented by the activity blocks 422b-422j respectively. In this example, the optional activities such as that of activity block 422c do not include notify and later input buttons, but alternatively, all activities could have the notify and later input buttons.

FIG. 4D shows a notification selection interface 440 that is displayed based on activating the notification button on any of the activity blocks displayed by the schedule interface 420 in FIG. 4C. The notification selection interface 440 displays a description 442 of the selected activity. A time field 444 allows a user to select a notification time for the onset of the activity. A day selection field 446 allows a user to select one or more days in a week or every day in the week for the notification to be displayed. Once the notification is set, the wearable device 150 will trigger an audio or visual alert to the user some predetermined time prior to or at the start time of the scheduled activity.

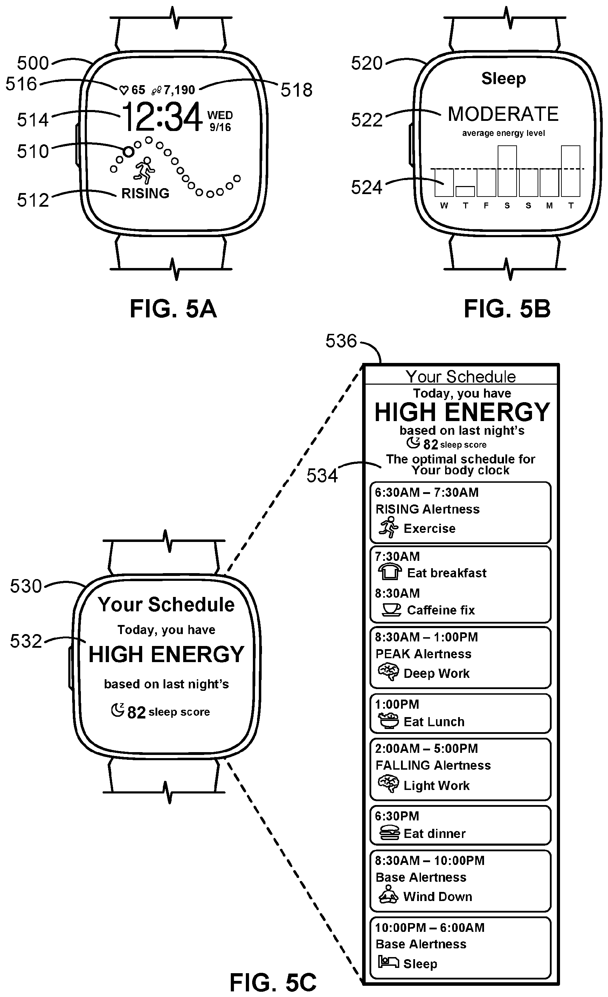

FIG. 5A shows a standard chronotype interface 500 that is displayed on the wearable device 150 by the wearable application 276. The interface 500 includes an alertness sinusoidal curve or waveform 510 that indicates the specific alertness cycle dictated by the chronotype of the user. As explained above, when the curve 510 slopes upward, alertness levels as dictated by the chronotype are increasing, while when the curve 510 slopes downward, alertness levels are decreased. The curve 510 thus allows a user to coordinate their activities with the alertness level as determined by their chronotype. In this example, an icon 512 representing user activity is displayed under the curve. In this example, the icon 512 may be one of a predetermined number of icons representing activities such as exercise, meals, and work as will be explained. Other graphics may be used such as the activity icon being plotted on the curve. The curve is depicted by dots, but the curve may use other graphics, textures, or colors to represent the alertness level at the time.

The interface 500 may also include conventional information generated by the data from the sensors of the wearable device 150 such as a time and date readout 514, a heartrate 516, a step counter 518, and other operational data such as the battery level. In this example, the clock face generated on the wearable device 150 may include different variations that may be set by the user. The variations include digital or analog output, whether the clock face remains on the display, different styles depending on the chronotype of the user, changes in interface depending on the time of the day, and a default interface if the user has not selected a chronotype.

Based on the alertness sinusoid, the wearable device 150 may be set to provide notifications and information for suggested activities based on the alertness curve 510. Throughout the day the user receives notifications and tips based on the time of the day in relation to the alertness curve 510. The notifications may include activities scheduled for optimal times, reminders to begin activities, reminders to stop activities, and the like that are tailored for the user.

Thus, the interface 500 may have different color codes for the alertness level. A first color such as orange may be used in either the background or the alertness sinusoid for high alertness. A second color such as purples may be used for falling or rising alertness period. A third color such as blue may be used for base (lowest) alertness. The activity icons may include a deep work icon, a light work icon, an exercise icon, a winddown icon, a sleep icon, a stimulant icon, breakfast icon, a lunch icon, and a dinner icon. The activity icons may be changed based on scheduling by the user as explained below or recommendations based on the point on the alertness curve 510.

FIG. 5B shows a sleep interface 520 that displays the energy level for each day based on the sleep of the user. The sleep score is used to indicate if the user should expect low, moderate or high energy for the day. The sleep interface 520 includes a description 522 of the energy level average. The energy level is a classification of how much energy the user can expect throughout the day based on the previous night sleep of the user. In this example, the energy level is based on a sleep score such as a FitBit sleep score (excellent or good sleep = high energy, fair sleep = moderate energy, and poor sleep = low energy). The sleep interface 520 also includes a bar chart 524 that shows the energy level average for each day of the week of the past seven days.

FIG. 5C shows a schedule interface 530 that displays an optimal schedule with different activities and corresponding alertness for the user based on their chronotype. The schedule interface 530 includes an energy level indication 532 of the energy level (high, low or moderate) from the sleep score. Thus, high energy reflects good sleep, moderate energy reflects fair sleep, and low energy reflects poor sleep. A schedule 534 that includes an ideal arrangement of activities such as that shown in FIG. 4C is shown. A scroll inset 536 shows the different activities that may be scrolled by the user to be viewed on the interface 520. Each of the activities may be displayed with a different color indicating different levels of alertness as well as the time period and description of the activity. Tapping on any of the displayed activities opens an activity screen, which gives the time and description of the activity. The activity screen shows which alertness level the activity corresponds to.

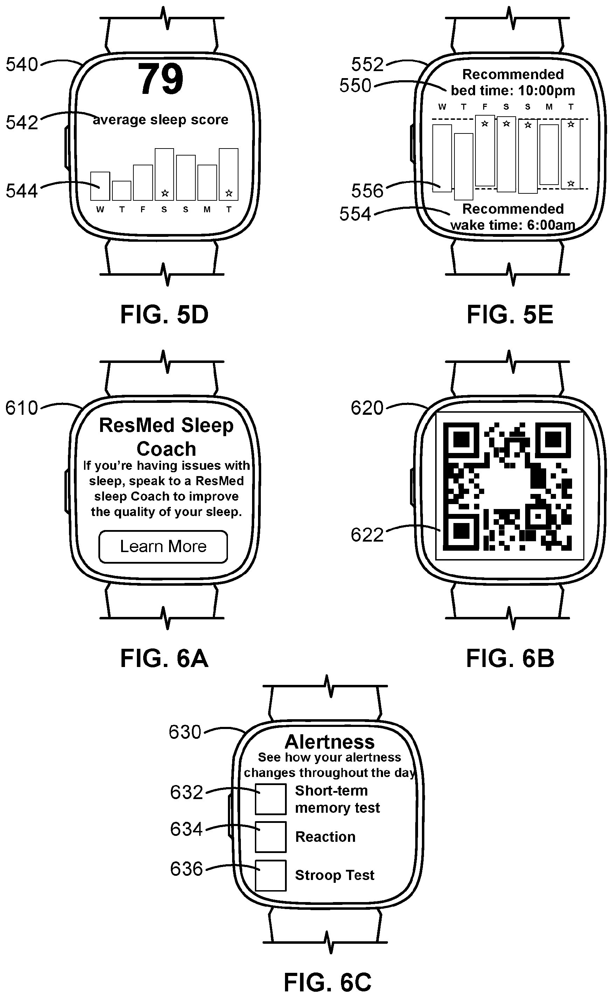

FIG. 5D shows a sleep score interface 540 that displays the specific sleep score for the user for each day. The interface 540 includes an average sleep score 542 and a graph of sleep scores 544 over a period of days. The graph 544 shows bars representing the sleep scores for the specific day. Certain days with the sleep scores that meet a desired sleep goal may be highlighted by a star symbol in this example.

FIG. 5E shows a wake time interface 550 shows the optimal wake times based on the selected chronotype and correlated research. The wake time interface 550 shows a recommended wake time 552 that is determined by the chronotype of the user. A recommended sleep time 554 is displayed that is determined by the chronotype of the user. A graph of wake times 556 over a period of days is also included on the interface 550. Certain days with the optimal wake up time may be highlighted by a star symbol in this example.

The wearable application 276 allows a user to determine information relating to different aspects in relation to the determined chronotype. FIG. 6A shows a sleep coaching interface 610 that shows information relating to sleep and accesses other resources. The sleep coaching interface 610 may be displayed by different inputs from other interfaces generated by the wearable application 276 such as the interfaces 540 and 550 in FIGs. 5D-5E.

FIG. 6B shows an interface 620 that displays a visual indicator such as a QR code 622 that allows access to a web portal that may provide further information to the user. The QR code 622 may be displayed based on certain interactions by the users, such as after a user finishes the questionnaire for determining chronotype and enters the specific chronotype as show in FIG. 4B. The user may show the wearable device 150 with the QR code 622 displayed in front of the camera on a web-enabled device such as the mobile device 130. The mobile device 130 will use the information in the QR code 622 to navigate the user to a webpage to provide information and answer any questions relating to the chronotype. The QR code 622 may also activate features on the webpage that allows the collected data from user answers to be sent to the wearable device 150 for the application 276 to make more detailed adjustments to the individual user.

The use of the QR code 622 simplifies the display of a website on another device through the wearable device 150. The QR code may be displayed by different inputs on the interfaces such as a call to action button displayed by the wearable interface. The QR code is used to refer the users to a variety of webpages that need to be viewed on web-viewer device. For example another QR code will display if a user would like to book an appointment with a sleep coach, resulting in the display of a website appointment booking page on the web-enabled device. Another QR code may result in the display of an ecommerce page that has personalized sleep products and tips tailored for the selected chronotype.

FIG. 6C shows an alertness testing interface 630 that allows a user to view how alertness changes throughout the day based on the chronotype. The alertness testing interface 630 includes different buttons that activate different tests relating to alertness. In this example, the testing interface 600 includes a short-term memory test button 632, a reaction time test button 634, and a Stroop test button 636. When a particular test button is selected, a specific test interface is displayed that allows the user to take the test to determine a parameter related to alertness. The interface will display average scores at peak alertness and base alertness. The interface will also display past scores and times that the tests that produced the scores were taken.

Thus, the short-term memory test may display a sequence for a user and ask the user to input the sequence that was displayed. The score is calculated based on how much of the sequence the user remembers. The reaction time test may be asking a user to hit a button when the screen changes color. The time to hit the button is then scored. The Stroop test asks a user to select a word that correlates with a described color and a score is determined on the number correct from a predetermined number of questions and the time to complete the test.

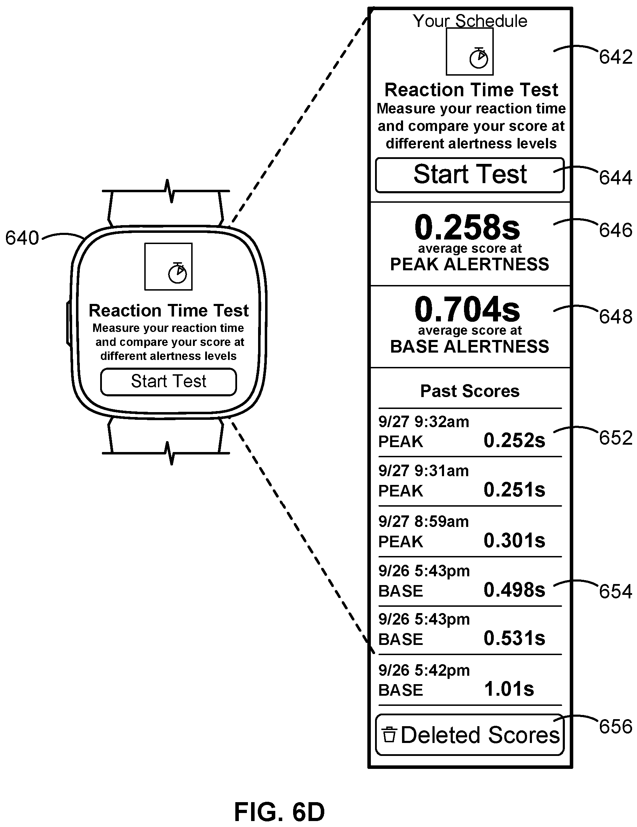

FIG. 6D shows an example reaction test summary interface 640 that is displayed when the reaction time test button 634 is selected in FIG. 6C. A similar summary interface may be shown for each of the other alertness tests. The reaction test summary interface 640 includes a summary information field 642 that explains the purpose of the reaction time test. The interface 640 includes a start test button 644 that activates a test interface. The interface 640 allows a user to scroll down and show an average score at the peak alertness level 646 and an average score at the base alertness level 648. The average scores are calculated from the past 10 scores in this example. The interface 640 also shows a listing of individual scores that is organized by scores at peak alertness 652 and scores at base alertness 654. The scores may be deleted by selecting a delete button 656.

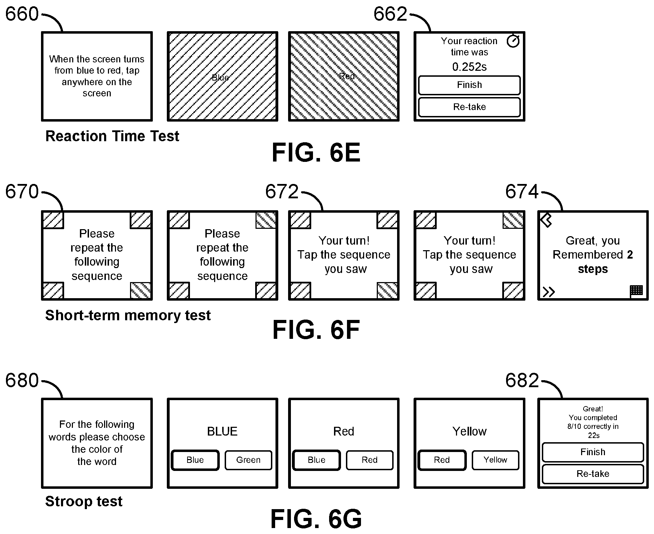

FIG. 6E shows a series of reaction test interfaces that are displayed when the start test button 644 in FIG. 6D is selected. An initial instruction interface 660 is displayed that instructs the user to tap the screen when the color of the interface changes from blue to red. After the user taps the screen, a results interface 662 is displayed showing the reaction time score. The results interface 662 includes a retake button that cycles back to the initial interface 660 and a finish button that loops the user to the test summary interface 640.

FIG. 6F shows a series of short-term memory test interfaces that are displayed when a start test button of a short-term memory test summary interface is displayed. An initial instruction interface 670 is displayed that instructs the user to repeat a sequence of a color displayed in one of the four corners. For example, three of the four corners may be colored blue while the fourth corner may be green. The sequence is displayed, and the user is instructed in a test screen 672 to tap the corners according to the sequence of the green corner was moved that was seen. After the user taps the sequence from memory, a results interface 674 is displayed showing memory result. The memory result score is based on how many steps in sequence the user remembered and was able to tap in the right order.

FIG. 6G shows a series of Stroop test interfaces that are displayed when a start test button of a Stroop test summary interface is displayed. An initial instruction interface 680 is displayed that instructs the user to choose the color of the word that is displayed. For example, the first panel has the word "Blue" colored in green. The second panel has the word "Red" colored in blue. The third panel has the word "Yellow" colored in yellow. After the user enters their choices on different testing screens, a results interface 682 is displayed showing the reaction time score. The results interface 682 includes a retake button that cycles back to the initial interface 680 and a finish button that loops the user to the Stroop test summary interface.

The above example displays and interfaces provide a user the understanding of their specific chronotype. This allows mapping of the user's individual own body clock to time periods during a day. Certain of the interfaces also allow the user to receive advice and direction to achieve optimal energy throughout the day based on their specific chronotype.

Although the present example determines a chronotype through a questionnaire answered by the user, other methods may be used to determine the chronotype of a user based on objective data. For example, sensor data from physiological sensors monitoring the user may be combined with data from other sensors that allow determination of activity times and sleep times. Thus, information on sleep and wake times, activity levels through the day, and heart rate data may be used to determine alertness levels throughout the day. The collected data may be analyzed to determine the chronotype of a user automatically according to a rule or previous analysis method. For example, the sleep/wake times, activities record, and heart rate may be used to plot an alertness curve that may be fit to a specific chronotype classification. Collected physiological data for chronotype determination may include heart rate variability (HRV), Oxygen saturation (SpO2 via pulse oximetry), heart rate (HR), and pulse rate in relation to times of the day for a user. Other collected data may include metrics such as electrodermal activity or cortisol levels (to measure stress) and light exposure. The physiological data may be correlated with alertness and used to determine the chronotype classification. Alternatively, a large data set from users with defined chronotypes may be used to train a machine learning model to determine the chronotype based on weighted data factors.

The alertness data collected by the wearable application 276 may be combined with other data related to the user to determine the chronotype. For example, if the user uses a respiratory therapy device such as a continuous positive airway pressure device (CPAP), operational data from the CPAP device such as the length and time of usage could be used as an input to the alertness level or score for the day. CPAP usage times could also be used to determine the chronotype as this data will identify sleep onset and awakening times. Operational data from other therapy devices such as Non-invasive ventilation (NIV) devices, invasive ventilation devices, portable oxygen concentrators (POCs), sleep apps may also be used to determine activity as well as sleep and wake times.

The wearable application 276 may also continue to collect relevant data as to the sleep patterns and activities of a user after the user selects a chronotype classification. Other data such as physiological data or operational data from therapy devices may also be collected and analyzed subsequent to the initial chronotype classification. As will be explained below, alertness data may be collected through the use of alertness tests such as a short-term memory test or a reaction test. The results of the alertness tests may be used to calibrate the alertness waveform for a specific user. The subsequent collected data may thus be analyzed periodically to determine whether the initial chronotype classification is accurate. The follow up collected data may be used to modify the body clock and corresponding notifications operated by the wearable application to further personalize the clock and thus shift the alertness waveform shape for the specific user. The corresponding notifications and tips may also be personalized further based on the subsequent follow up data. Alternatively, if the data indicates that the current chronotype classification is incorrect, options (such as tips) can be offered to the user via the wearable application 276 to correct the classification.

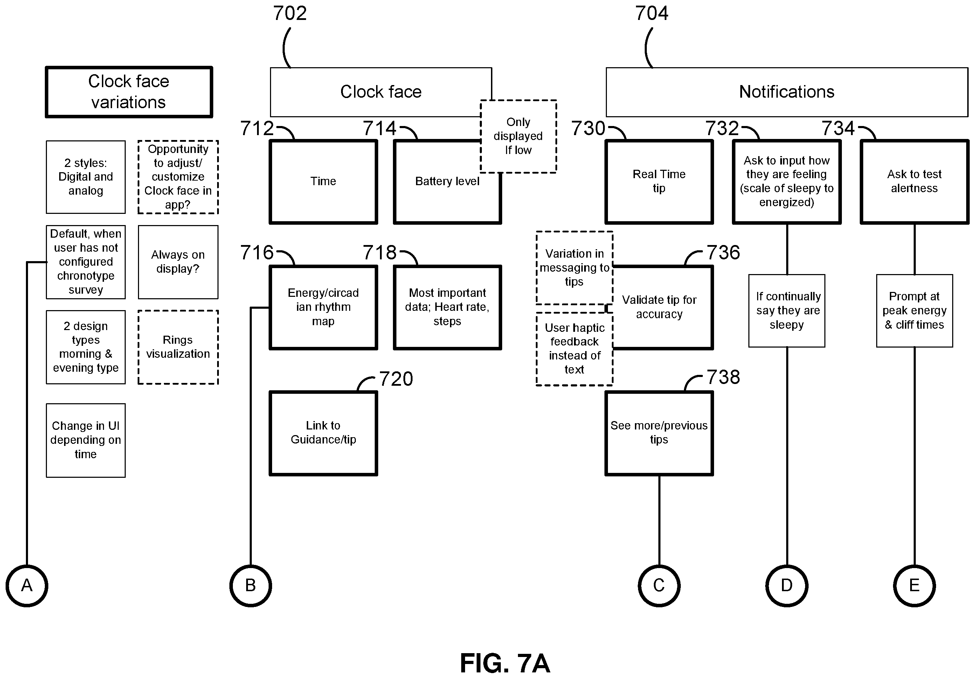

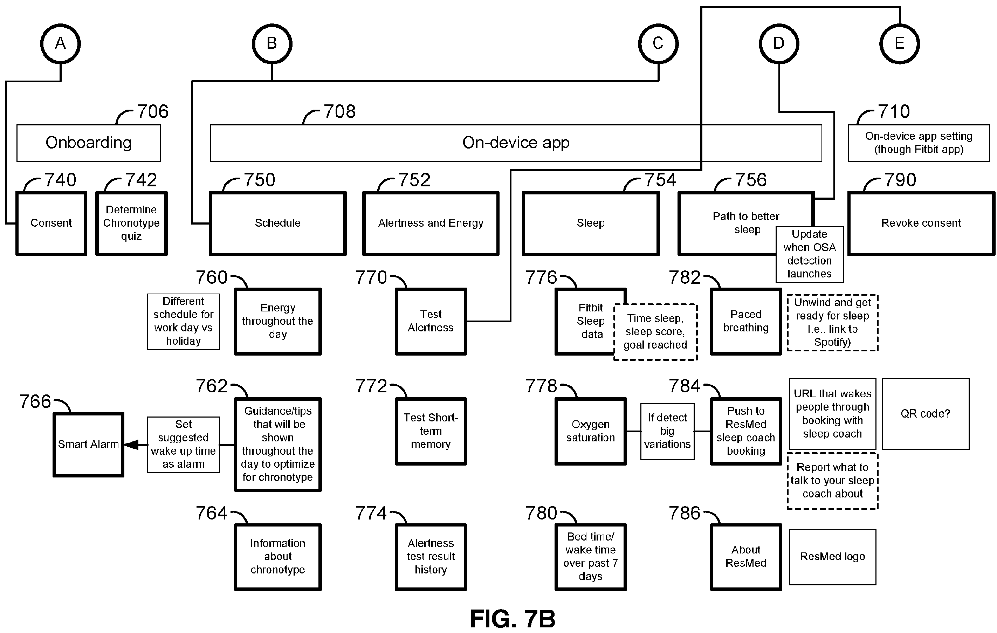

FIGs. 7A-7B is a state diagram 700 of the different functions of the wearable application 276 on the wearable device 150. In this example, there are several main interface states for the wearable device 150 including a clock face interface state 702, a notifications interface state 704, an onboarding interface state 706, an application functions interface state 708 and a coordination interface state 710.

The clock face interface state 702 includes a time output 712, a battery level output 714, an energy/circadian rhythm map output 716, a physiological data output 718 and a links output 720. As explained above, the clock face generated on the wearable device 150 may include different variations that may be set by the user.

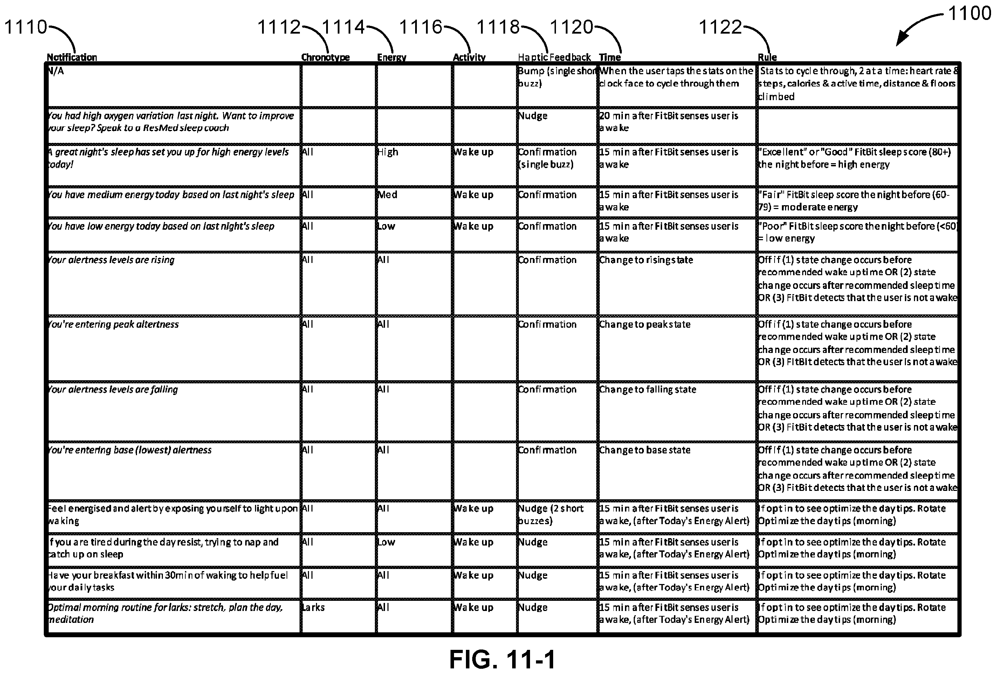

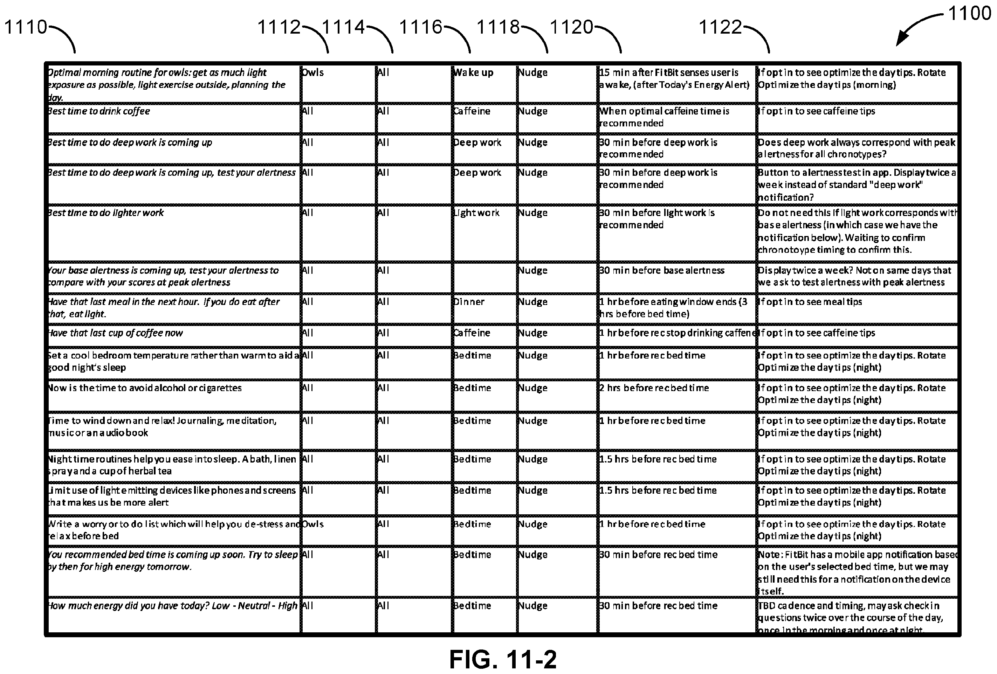

The notifications interface state 704 includes a real time tip output 730, an input for determining tiredness 732, an alertness test input 734, a validation output 736 and a see more tips output 738. Example notifications are listed in the table in FIG. 11. The real time tip output 730 may be activated from the links output 720.

The onboarding interface state 706 includes a consent output 740, and a determine chronotype questionnaire output 742. As explained above, the questionnaire may be accessed on a webpage or internally using the wearable device 150. The consent output 740 may include a link to a privacy policy or a consent application for collection of data from the user. The clock face 702 will include an input that displays the consent output 740 when the wearable application 276 is first set up.

The on-device application interface state 708 includes a schedule output 750, an alertness and energy output 752, a sleep output 754, and a sleep suggestion output 756. The schedule output 750 includes an energy throughout the day output 760, a tips output 762, and a chronotype information output 764. FIG. 5C shows an example interface of the schedule output 750. The schedule output 750 may include different schedules, such as a weekday, weekend, or holiday. The recommended chronotype schedule may be more flexible for people who are shift workers or who have sudden lifestyle changes such as going on vacation. The schedule output 750 may be accessed from the energy rhythm map output 716 or the see more tips output 738. The energy throughout the day output 760 shows the sinusoid alertness curve 510 in FIG. 5A. The alertness and energy levels may be interposed over the scheduled activity. The tips output 762 accesses various tips at different types to indicate times for optimizing activities specific to the chronotype. An optional alarm 766 may be set to trigger an audio or visual indicator on the wearable device 150. A smart alarm may wake a user during a specific sleep stage that may help the user more awake and alert in the morning. The chronotype information output 764 shows information about the chronotype of the user.

The alertness and energy output 752 includes a test alertness output 770, a test short-term memory output 772, and an alertness test history output 774. FIGs. 6D-6G show examples of the test interfaces that incorporate the test outputs. The test alertness output 770 may be accessed from the ask to test alertness output 734 of the notifications interface 704.

The sleep output 754 includes a sleep data output 776, an oxygen saturation output 778, and a sleep times output 780. The sleep data output 776 shows data such as the amount of time slept for a day, a sleep score and whether sleep goals have been achieved. The oxygen saturation output 778 shows oxygen saturation levels. The oxygen saturation levels are determined by a wearable device with an appropriate sensor. The sleep times output 780 shows the bed time and wake time over a predetermined period such as over the past 7 days.

The sleep suggestion output 756 includes a paced breathing output 782, a push to outside professional 784, and a health care provider information output 786. These outputs are updated when an obstructive sleep apnea (OSA) detection feature is launched from the wearable device 150. The paced breathing output 782 may display a link to play soothing music or activate another device such as the built in paced breathing features of a smart watch. For example, paced breathing can also be achieved by instructing a user to breath in time following an on-screen animation or a light fading in/out. As explained above, the push to outside professional output 784 displays a QR code that allows access to a web portal for an audio or video consultation. When the portal is activated, a summary report of any concerning sleep data detected (e.g. graph of overall oxygen saturation levels, and graphs of sleep data and bed/wake times over the past week) may be made available to either the user or the outside professional or both. The push to outside professional output 784 may be activated from the oxygen saturation output 778 if large variations in oxygen saturation are detected, indicating potential sleep problems. The push to outside professional output 784 may be activated if the response to the input for determining tiredness 732 continuously indicates the user is sleepy. The health care provider information output 786 displays information about the wearable application provider such as the logo of the provider.

The on device settings interface 710 includes a revoke consent output 790 that allows a user to revoke the consent to access their data and information.

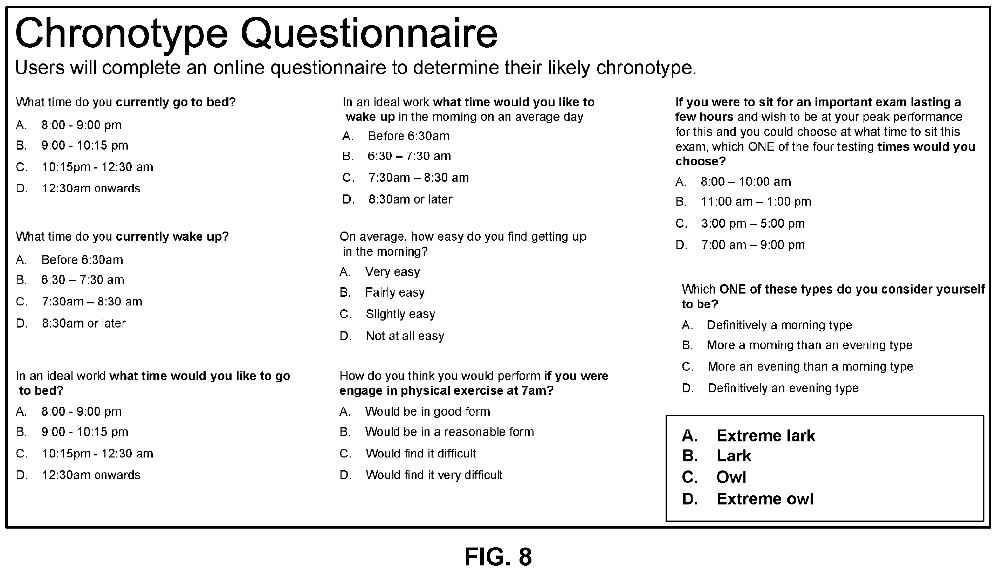

FIG. 8 shows an example questionnaire that allows determination of a chronotype that is displayed on a website interface. As shown in the questionnaire 800, the user may select a bed time, wake time, ideal time to go to bed, ideal time to wake up, ease of waking up, performance of exercise, mental exam, and a subjective category of person type. The user may be characterized as one of four chronotypes (extreme lark, lark, owl, extreme owl) based on the answers to the questions in FIG. 8. In this example, there are 4 multiple choice options with each question and each of these 4 options is given a 1 weighting: A (extreme lark), B (lark), C (owl), D (extreme owl). The majority picked determines chronotype. The content of the questions in FIG. 8 is important to determination of the chronotype classification.

The results for the questionnaire in FIG. 8 may be stored by the application 276 as well as in the external database 160 that includes other relevant demographic and medical data for the user. The wearable application 276 and or applications on the mobile device 130 in FIG. 1 may access the database 160 to further tailor individual information and tips for the user.

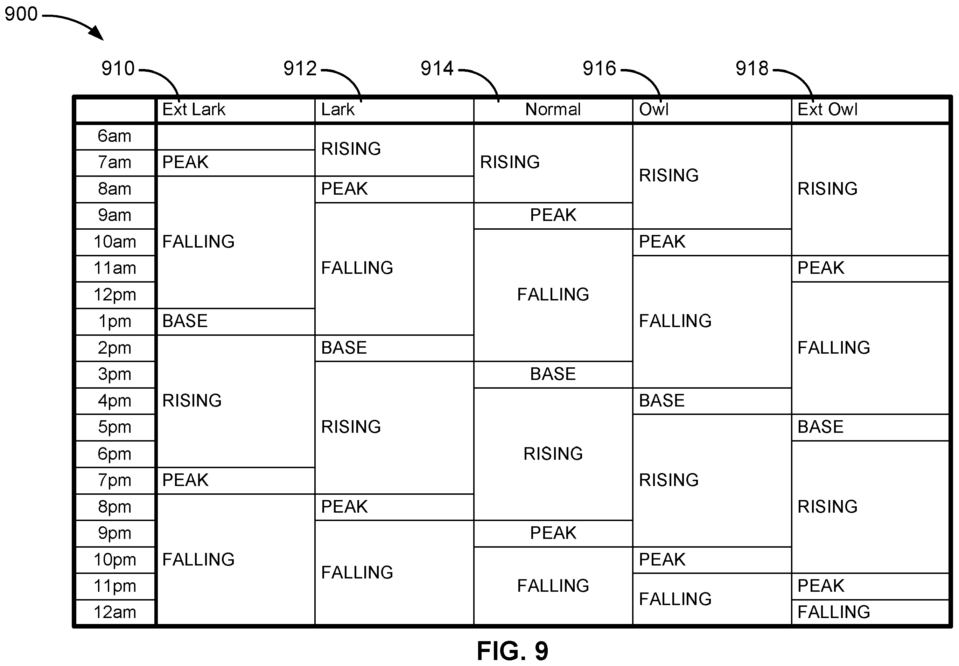

FIG. 9 is a chart 900 that shows different alertness levels by times according to five different chronotypes. The chart 900 has a column for extreme lark 910, a column for lark 912, a column for normal 914, a column for owl 916, and a column for extreme owl 918. Each of the chronotypes has a corresponding column 910, 912, 916, and 918 having different times for peak periods, rising periods, base periods, and falling periods. The mean values between owls and larks are in the normal column 914. As shown in FIG. 9, extreme larks get most of their energy in very early times of the morning while larks get most of their energy approx. an hour later than extreme larks. Owls have more energy later in the day and the early evening, extreme owls get most of their energy an hour or so later than owls well into the late evening. The alertness sinusoidal wave corresponds to the rising and falling times on the chart as well as peak and base energy times.

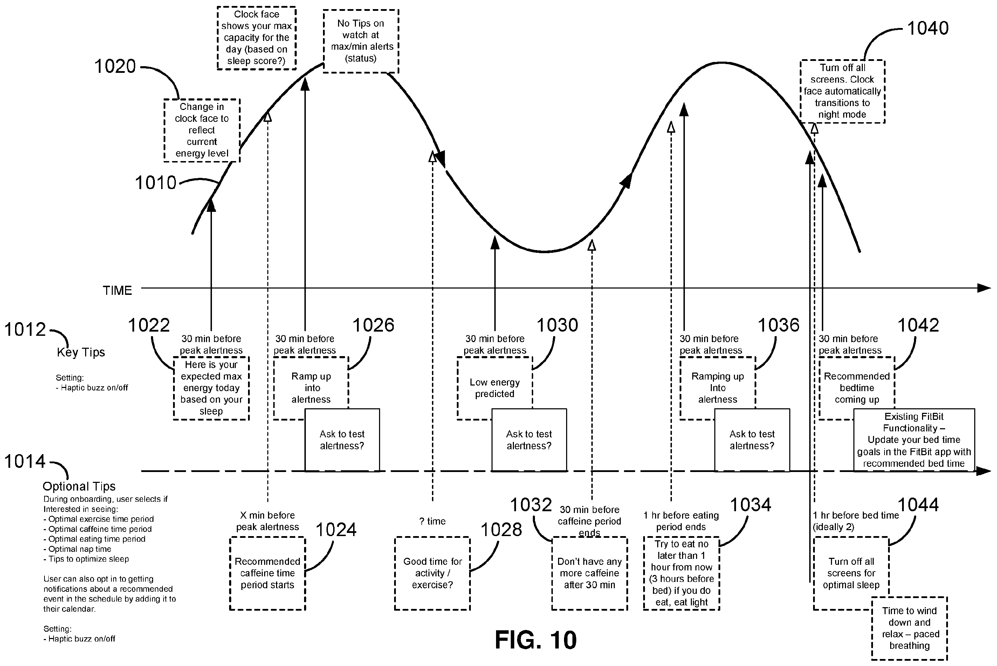

FIG. 10 is an example of different tips that may be displayed on the wearable device based on the curve for the individual. The chart in FIG. 10 shows an example curve 1010 plotted over time, and a set of key tips 1012, and a set of optional tips 1014. The relation between the curve 1010 and time periods is set based on the chronotype of the user. In this example, the key tips 1012 relate to alerts relating to energy level. During onboarding, a user may select some or all of the optional tips 1014 that may be displayed in relation to optimal exercise periods, optimal stimulant periods, optimal easting periods, optimal nap times, tips relating to sleep. Other example activities may also be generated in the form of tips that may be displayed on selection of the user during optical times based on the user chronotype. The wearable application 276 may receive notifications, or allow addition to an on-board calendar.

In this example, the curve 1010 initially increases indicating an increase in energy level for the user. The curve 1010 starts on an upward slope from the time a user wakes up. During the upward slope, the wearable device 150 changes the interface to reflect graphics showing the current energy level is increasing (1020). A key tip relating to an indication of maximum energy based on the chronotype is displayed 30 minutes after waking up in this example (1022). The display of the wearable device 150 may show the maximum capacity for the day for the user near the time of the peak of the curve 1100. The maximum capacity may be determined from a sleep score determined from a built-in algorithm on the example wearable device 150. A set time period before the estimated maximum energy level in the curve 1010, an optional tip relating to recommending taking a stimulant (such as caffeine) may be displayed (1024). In this example, 30 minutes before the estimated peak energy level, another tip indicating the user is ramping up to maximum alertness is displayed (1026). The display may also ask the user to take an alertness test. The alertness test results allow the user to confirm the right chronotype as correlating the test results with the predicted times for peak alertness. This is also so that the user can set a benchmark score at peak alertness, so s/he can see for him/herself how the score differs at different alertness levels.

After maximum energy, the curve 1010 begins a downslope. During the downslope, an optional tip relating to exercise or an activity may be displayed (1028). In this example, 30 minutes before a low energy point on the curve 1010, a key tip predicting low energy is displayed (1030). The display may also ask the user to take an alertness test. After the low energy point is reached, the curve 1010 begins an upslope. The period for allowing stimulants to be taken ends on this upslope and thus an optional tip warning that no stimulants should be taken is displayed 30 minutes before the period ends (1032). This allows a user to maximize sleep effectiveness. Similarly, an optional tip warning that eating should not occur is displayed 60 minutes before the eating period ends (1034). This also allows a user to maximize sleep effectiveness. During the upslope, a key tip is displayed indicating the user is ramping up to maximum alertness is displayed (1036). The display may also ask the user to take an alertness test.

The curve 1010 reaches a second peak and then starts on another downslope. During the downslope, the user should be ready to go to sleep. Thus, the display of the wearable device 150 may be set to transition to night mode (1040). During this time, an optional tip may be displayed an hour before bedtime, such as recommended that all screens are turned off, or to wind down and relax (1044). A key tip is displayed indicating that it is 30 minutes before bedtime (1044).

FIG. 11 is a table of notifications 1100 that may be available for display on the interfaces of the example wearable device 150. The table 1100 has a column of the notifications 1110. Each of the notifications may depend on the chronotype as indicated by a chronotype column 1112. Certain notifications may be displayed for all chronotypes. The notifications may also be displayed depending on the energy level as displayed in an energy column 1114. Certain notifications may be displayed for all energy levels. The notifications may be attached to certain activities such as wake up, caffeine, deep work, light work, dinner, or bedtime as shown in an activities column 1116.

Each notification is associated with a particular haptic feedback, such as a buzz, confirmation, or a nudge as shown in a haptic feedback column 1118. For example, depending on the type of notification the user will be alerted through a single short buzz, a single long buzz, 2 short buzzes or an on screen notification. A time column 1120 indicates the time that a notification will be initiated. Certain notifications are made based on a user input, other notifications are initiated based on a predetermined period of time before or after an event such as waking up, other notifications are initiated based on changes in the alertness curve. A rules column 1122 indicates the conditions that the notification is displayed.

The example notifications in the table 1100 may include notifications of the level of energy after sleep such as high, moderate or low energy levels based on the sleep quality determined by the wearable device 150. The notifications may also include when the alertness of a user based on whether their alertness sinusoid is rising, at a peak, falling or at a low. The notifications may also include different suggestions for activities based on the time of the day and the chronotype of the user. There may also be notifications that are tied to alertness that may suggest different tests such as alertness tests. There may also be notifications related to either night time or bed time. These notifications may include suggestions of when to eat, when to have stimulants, minimizing stressful activities, and tips for good sleep.

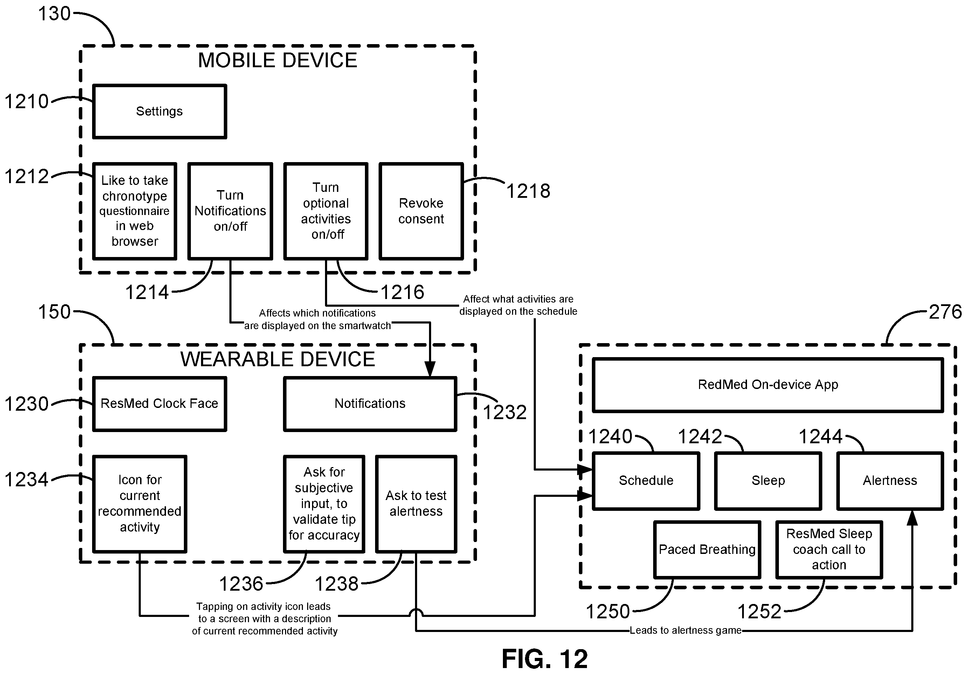

FIG. 12 is a block diagram showing the interactions between the wearable device 150, the wearable application 276, and the mobile device 130 in FIGs. 1 and 2. The wearable device 150 is communicatively linked to the mobile device 130 as explained above. The mobile device 130 includes a settings state 1210. The settings state 1210 includes a questionnaire link 1212 that displays the questionnaire in FIG. 8. The settings state 1210 includes a notifications control 1214 that allows a user to control the notifications on the wearable device 150. The settings state 1210 includes an activities control 1216 that allows the user to control optional activities displayed by the wearable application 276. The settings state 1210 includes a revoke consent control 1218 that allows a user to control their consent for information available to the mobile device 130 and the wearable application 276.

The wearable device 150 may either be in a clock face display state 1230 or a notifications state 1232. The clock face display state 1230 generally displays the alertness sinusoid as shown in FIG. 5A. The clock face display state 1230 accesses a recommended activity icon input 1234. The accessing of the activity icon input 1234 allows for the display of an interface with a description of the current activity. The notifications state 1232 may be set by the mobile device 130 through the settings state 1210. The notifications state 1232 allows the display of a subjective input 1236, and a test alertness input 1238. The subject input 1236 allows the display of different tips. The test alertness input 1238 allows the user to run the different alertness tests as shown in FIGs. 6D-6G above.

The wearable application 276 has a schedule state 1240, a sleep state 1242, and an alertness testing state 1244. The schedule state 1240 displays the activities selected by a user via the activities control 1216 in a schedule display as shown in FIG. 5C. The sleep state 1242 allows the user to see sleep and energy data, access a paced breathing program 1250 to wind down and prepare for sleep, and find information 1252 on booking a session with a professional such as a sleep coach. The alertness test state 1244 allows the user to access the interfaces to test alertness.

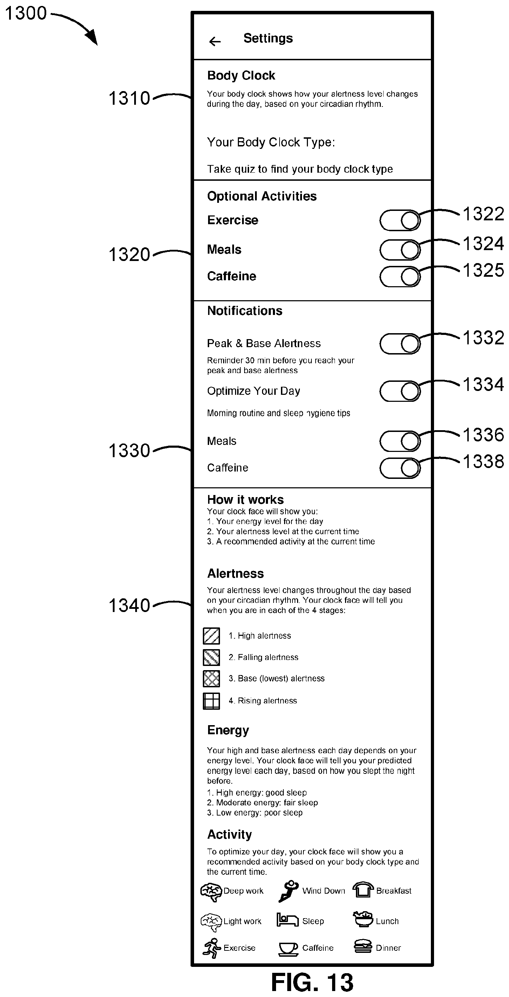

FIG. 13 is a screen image of an example settings interface 1300 for the mobile device 130 that is linked to the wearable application 276. The parts of the screen image 1300 may be displayed by a user scrolling the display of the wearable device 150. The settings interface 1300 includes a body clock section 1310 that includes a link to display the questionnaire in FIG. 8 on the mobile device. An optional activities section 1320 includes an exercise toggle switch 1322, a meals toggle switch 1324, and a stimulant toggle switch 1326. The toggle switches 1322, 1324, and 1326 allow a user to activate or deactivate such activities for purposes of scheduling.

A notifications section 1330 includes a peak and base alertness toggle switch 1332, an optimization toggle switch 1334, a meals toggle switch 1336, and a stimulant toggle switch 1338. The notifications toggle switches 1332, 1334, 1336, and 1338 allow a user to show the notifications that are selected. Thus, a user may be alerted of peak and base alertness, tips during optimal times to perform activities, and times to have meals or take stimulants like caffeine. An explanation section 1340 includes information on the notifications and the display interfaces of the wearable application 276.

Physiological measurements taken from the wearable device 150 coupled with operational data from a therapy device such as a CPAP device may be used for other purposes. For example, the operational data for a CPAP device and residual Apnea Hypopnea Index (AHI) data can be used to show improvements in alertness due to CPAP therapy to the user. These improvements may be displayed to the user on the wearable device 150 or a personal mobile device 130. These improvements could also feed into the algorithm of the wearable application 276 that determines the optimal time for the user to performance certain activities. This information could therefore serve as additional motivation for a user to comply with CPAP or other therapy regimes.

As explained above a machine learning model may be trained to automatically determine a chronotype for a user based on input factors that may include demographics, activities, and sleep data. The machine learning model may also be used to refine the time correlation with a particular initial sinusoid to further refine the sinusoid individually more accurately to a specific individual body clock.

The collection of data from a user population may be used to train a machine learning model to correlate input factors to specific chronotypes as well as determine a specific sinusoid for each user. The inputs for the machine learning model may include a variety of data that may be evaluated in the model design process. The machine learning model is trained and internal weights are adjusted based on the training data set. After evaluation against the training set, the model may be deployed after reaching a predetermined accuracy level.

In this example the machine learning model is a neural network. The neural network may be a multilayer perceptron (MLP) neural network model with no direct connections between nodes and the use of one or more hidden layers. The neural network MLP model adjusts internally derived calculated weights between each of the established node connections by minimizing an error function against actual values during the training process. Other examples of machine learning models may include a decision tree ensemble, a support vector machine, a Bayesian network, or a gradient boosting machine. Such structures can be configured to implement either linear or non-linear predictive models.

Unsupervised machine learning may also be used to discover additional correlations between data and chronotype. Machine learning may employ techniques such as neural networks, clustering or traditional regression techniques. The training data may be used to test different types of machine learning algorithms for the machine learning and determine which one has the best accuracy in relation to predicting chronotype. The machine learning model may be continuously updated by new input data from the system in FIG. 1. Thus, the model may become more accurate with greater data collection by the system 100.

As used in this application, the terms "component," "module," "system," or the like, generally refer to a computer-related entity, either hardware (e.g., a circuit), a combination of hardware and software, software, or an entity related to an operational machine with one or more specific functionalities. For example, a component may be, but is not limited to being, a process running on a processor (e.g., digital signal processor), a processor, an object, an executable, a thread of execution, a program, and/or a computer. By way of illustration, both an application running on a controller, as well as the controller, can be a component. One or more components may reside within a process and/or thread of execution, and a component may be localized on one computer and/or distributed between two or more computers. Further, a "device" can come in the form of specially designed hardware; generalized hardware made specialized by the execution of software thereon that enables the hardware to perform specific function; software stored on a computer-readable medium; or a combination thereof.

The terminology used herein is for the purpose of describing particular embodiments only, and is not intended to be limiting of the invention. As used herein, the singular forms "a," "an," and "the" are intended to include the plural forms as well, unless the context clearly indicates otherwise. Furthermore, to the extent that the terms "including," "includes," "having," "has," "with," or variants thereof, are used in either the detailed description and/or the claims, such terms are intended to be inclusive in a manner similar to the term "comprising."

Unless otherwise defined, all terms (including technical and scientific terms) used herein have the same meaning as commonly understood by one of ordinary skill in the art. Furthermore, terms, such as those defined in commonly used dictionaries, should be interpreted as having a meaning that is consistent with their meaning in the context of the relevant art, and will not be interpreted in an idealized or overly formal sense unless expressly so defined herein.

One or more elements or aspects or steps, or any portion(s) thereof, from one or more of any of claims 1 to 59 below can be combined with one or more elements or aspects or steps, or any portion(s) thereof, from one or more of any of the other claims 1 to 59 or combinations thereof, to form one or more additional implementations and/or claims of the present disclosure.

## Claims

1. A method to display alertness to a user, the method comprising:
determining a chronotype classification for the user;
aligning an alertness waveform associated with alertness levels at different times based on the chronotype classification;
displaying the alertness levels from the alertness waveform to the user on a display (280) of a wearable device (150);
displaying an alertness test on the wearable device (150);
receiving a result of the alertness test from the user;
correlating the result of the alertness test with an alertness level determined from the alertness waveform; and
determining an average alertness score based on the result of a series of alertness tests taken at either a peak point or a base point of the alertness waveform.

2. The method of claim 1, further comprising displaying notifications for optimal activities at a predetermined time based on the chronotype classification; and/or
wherein the notifications include icons corresponding to one of the optimal activities displayed on the display (280); and/or
wherein the method further comprises displaying a schedule interface (420; 530) displaying a schedule of activities for the user that includes an input to allow the user insert an optimal activity into the schedule of activities.

3. The method of any one of claims 1-2, further comprising displaying a notification for an optimal sleep time based on the chronotype classification.

4. The method of any one of claims 1-3, wherein the chronotype is determined based on the user answering questions from a questionnaire.

5. The method of any one of claims 1-4, wherein the chronotype is determined based on physiological data measured from the user; and/or
wherein the physiological data is determined by a physiological sensor on the wearable device (150); and/or
wherein the physiological data is analyzed to determine sleep and wake times, and activity levels through a day to determine the chronotype; and/or
wherein operational data from a therapy device is analyzed to determine sleep and wake times, and activity levels through a day to determine the chronotype.

6. The method of any one of claims 1-5, wherein the determination is performed with a machine learning model trained with data collected from a user population and the chronotype of each of the user population.

7. The method of any one of claims 1-6, further comprising displaying notifications to eat or take stimulants based on the chronotype; and/or
wherein the method further comprises displaying notifications to exercise or work based on the chronotype; and/or
wherein the method further comprises measuring sleep related data from the user and displaying a predicted energy level based on the measured sleep related data; and/or
wherein the sleep related data is a sleep score for the user.

8. The method of any one of claims 1-7, further comprising displaying a visual indicator (416; 620) on the wearable device (150) that enables the display of a website on a web-enabled device scanning the visual indicator; and/or
wherein the visual indicator is a QR code (622); and/or
wherein the website is one of a virtual coach website, an information website, or an instruction website.

9. The method of any one of claims 1-8, wherein the wearable device (150) includes a transceiver (266) that allows communication with a mobile device (150); and/or wherein the wearable device (150) is wrist-mounted.

10. The method of any one of claims 1-9, wherein a graphical representation (500) displayed on the display (280) of the wearable device (150) changes based on the time period and the alertness waveform; and/or
wherein the alertness levels are displayed in correlation to predetermined colors on the display (280).

11. The method of any one of claims 1-10, further comprising:
collecting additional data relating to the chronotype of the user subsequent to the chronotype classification; and
adjusting the alertness waveform based on the collected data.

12. The method of any one of claims 1-11, further comprising alerting a user of changes in alertness level based on the alertness waveform.

13. The method of any one of claims 1-12, further comprising:
displaying an alertness test on the wearable device;
receiving a result of the alertness test from the user; and
correlating the result of the alertness test with an alertness level determined from the alertness waveform; and/or
wherein the alertness test is one of a short-term memory test, a reaction test, or a Stroop test; and/or
wherein the method further comprises adjusting the alertness waveform based on the result of the alertness test.

14. A computer program product comprising instructions which, when executed by a computer, cause the computer to carry out the method of any one of claims 1 to 13.

15. A wearable device (150) allowing the display of alertness for a user, the wearable device (150) comprising:
a display (280);
a controller (260) coupled to the display (280), the controller (260) operable to:
read an input of a chronotype classification of the user;
align an alertness waveform relating to alertness of the user relative to time periods based on the chronotype classification;
display the alertness waveform on the display (280);
display an alertness test on the display (280);
receive a result of the alertness test from the user;
correlate the result of the alertness test with an alertness level determined from the alertness waveform; and
determine an average alertness score based on the result of a series of alertness tests taken at either a peak or base points of the alertness waveform.

## Patentansprüche

1. Verfahren zur Anzeige von Aufmerksamkeit eines Benutzers, wobei das Verfahren Folgendes umfasst:
Bestimmen einer Chronotyp-Klassifizierung für den Benutzer;
Ausrichten einer Aufmerksamkeitswellenform, die mit Aufmerksamkeitsniveaus zu verschiedenen Zeitpunkten verbunden ist, auf der Grundlage der Chronotypklassifizierung;
Anzeigen der Aufmerksamkeitsniveaus aus der Aufmerksamkeitswellenform für den Benutzer auf einem Display (280) einer tragbaren Vorrichtung (150);
Anzeigen eines Aufmerksamkeitstests auf der tragbaren Vorrichtung (150);
Empfangen eines Ergebnisses des Aufmerksamkeitstests vom Benutzer;
Korrelieren des Ergebnisses des Aufmerksamkeitstests mit einem anhand der Aufmerksamkeitswellenform bestimmten Aufmerksamkeitsniveau; und
Bestimmen eines durchschnittlichen Aufmerksamkeitswerts auf der Grundlage des Ergebnisses einer Reihe von Aufmerksamkeitstests, die entweder an einem Spitzenpunkt oder an einem Basispunkt der Aufmerksamkeitswellenform durchgeführt wurden.

2. Verfahren nach Anspruch 1, weiter umfassend Anzeigen von Benachrichtigungen über optimale Aktivitäten zu einem vorbestimmten Zeitpunkt auf der Grundlage der Chronotypklassifizierung; und/oder
wobei die Benachrichtigungen Symbole beinhalten, die einer der auf der Anzeige (280) angezeigten optimalen Aktivitäten entsprechen; und/oder
wobei das Verfahren weiter Anzeigen einer Zeitplanschnittstelle (420; 530) umfasst, die dem Benutzer einen Zeitplan von Aktivitäten anzeigt und eine Eingabemöglichkeit beinhaltet, mit der der Benutzer eine optimale Aktivität in den Zeitplan von Aktivitäten einfügen kann.

3. Verfahren nach einem der Ansprüche 1 oder 2, weiter umfassend Anzeigen einer Benachrichtigung über eine optimale Schlafenszeit auf der Grundlage der Chronotypklassifizierung.

4. Verfahren nach einem der Ansprüche 1-3, wobei der Chronotyp auf der Grundlage einer Beantwortung von Fragen aus einem Fragebogen durch den Benutzer bestimmt wird.

5. Verfahren nach einem der Ansprüche 1-4, wobei der Chronotyp auf der Grundlage physiologischer Daten, die von dem Benutzer gemessen werden, bestimmt wird; und/oder
wobei die physiologischen Daten durch einen physiologischen Sensor auf der tragbaren Vorrichtung (150) bestimmt werden; und/oder
wobei die physiologischen Daten analysiert werden, um Schlaf- und Wachzeiten sowie Aktivitätsniveaus im Tagesverlauf zu bestimmen, um den Chronotyp zu bestimmen; und/oder
wobei Betriebsdaten einer Therapievorrichtung analysiert werden, um Schlaf- und Wachzeiten sowie Aktivitätsniveaus im Tagesverlauf zu bestimmen, um den Chronotyp zu bestimmen.

6. Verfahren nach einem der Ansprüche 1-5, wobei die Bestimmung mit einem maschinellen Lernmodell durchgeführt wird, das mit Daten, die von einer Benutzerpopulation erhoben wurden, und mit dem Chronotyp jedes Benutzers trainiert wurde.

7. Verfahren nach einem der Ansprüche 1-6, weiter umfassend Anzeigen von Benachrichtigungen, Stimulanzien auf der Grundlage des Chronotyps zu essen oder einzunehmen; und/oder
wobei das Verfahren weiter Anzeigen von Benachrichtigungen zur sportlichen Betätigung oder zur Arbeit auf der Grundlage des Chronotyps umfasst; und/oder
wobei das Verfahren weiter Messen von schlafbezogenen Daten des Benutzers und Anzeigen eines auf der Grundlage der gemessenen schlafbezogenen Daten vorhergesagten Energieniveaus umfasst; und/oder
wobei die schlafbezogenen Daten einen Schlafwert für den Benutzer darstellen.

8. Verfahren nach einem der Ansprüche 1-7, weiter umfassend Anzeigen eines visuellen Indikators (416; 620) auf der tragbaren Vorrichtung (150), der die Anzeige einer Website auf einer internetfähigen Vorrichtung ermöglicht, die den visuellen Indikator scannt; und/oder
wobei es sich bei dem visuellen Indikator um einen QR-Code (622) handelt; und/oder
wobei es sich bei der Website um eine virtuelle Trainer-Website, eine Informations-Website oder eine Schulungs-Website handelt.

9. Verfahren nach einem der Ansprüche 1-8, wobei die tragbare Vorrichtung (150) einen Transceiver (266) beinhaltet, der Kommunikation mit einer Mobilvorrichtung (150) ermöglicht; und/oder wobei die tragbare Vorrichtung (150) am Handgelenk befestigt ist.

10. Verfahren nach einem der Ansprüche 1-9, wobei sich eine grafische Darstellung (500), die auf der Anzeige (280) der tragbaren Vorrichtung (150) angezeigt wird, in Abhängigkeit von der Zeitperiode und der Aufmerksamkeitswellenform ändert; und/oder
wobei die Aufmerksamkeitsniveaus in Korrelation zu vorbestimmten Farben auf der Anzeige (280) angezeigt werden.

11. Verfahren nach einem der Ansprüche 1-10, weiter umfassend:
Erheben zusätzlicher Daten, die sich auf den Chronotyp des Nutzers beziehen, im Anschluss an die Chronotypklassifizierung; und
Anpassen der Aufmerksamkeitswellenform auf Basis der erhobenen Daten.

12. Verfahren nach einem der Ansprüche 1-11, weiter umfassend Benachrichtigen eines Benutzers über Änderungen im Aufmerksamkeitsniveau auf der Grundlage der Aufmerksamkeitswellenform.

13. Verfahren nach einem der Ansprüche 1-12, weiter umfassend:
Anzeigen eines Aufmerksamkeitstests auf der tragbaren Vorrichtung;
Empfangen eines Ergebnisses des Aufmerksamkeitstests vom Benutzer; und
Korrelieren des Ergebnisses des Aufmerksamkeitstests mit einem anhand der Aufmerksamkeitswellenform bestimmten Aufmerksamkeitsniveau; und/oder
wobei der Aufmerksamkeitstest entweder ein Kurzzeitgedächtnistest, ein Reaktionstest oder ein Stroop-Test ist; und/oder
wobei das Verfahren weiter Anpassen der Aufmerksamkeitswellenform auf der Grundlage des Ergebnisses des Aufmerksamkeitstests umfasst.

14. Computerprogrammprodukt, umfassend Anweisungen, die bei Ausführung durch einen Computer den Computer zum Ausführen des Verfahrens nach einem der Ansprüche 1 bis 13 veranlassen.

15. Tragbare Vorrichtung (150), die Anzeigen einer Aufmerksamkeit eines Benutzers ermöglicht, wobei die tragbare Vorrichtung (150) Folgendes umfasst:
eine Anzeige (280);
eine mit der Anzeige (280) gekoppelte Steuereinheit (260), wobei die Steuereinheit (260) betriebsfähig ist, um:
die Chronotypklassifizierung des Benutzers einzulesen;
eine Aufmerksamkeitskurve, die sich auf Aufmerksamkeit des Benutzers bezieht, relativ zu Zeiträumen auf der Grundlage der Chronotypklassifizierung auszurichten;
die Aufmerksamkeitswellenform auf der Anzeige (280) anzuzeigen;
einen Aufmerksamkeitstest auf der Anzeige (280) anzuzeigen;
ein Ergebnis des Aufmerksamkeitstests vom Benutzer zu empfangen;
das Ergebnis des Aufmerksamkeitstests mit einem anhand der Aufmerksamkeitswellenform bestimmten Aufmerksamkeitsniveau zu korrelieren; und
einen durchschnittlichen Aufmerksamkeitswert auf der Grundlage des Ergebnisses einer Reihe von Aufmerksamkeitstests zu bestimmen, die entweder an einem Höhepunkt oder einem Tiefpunkt der Aufmerksamkeitswellenform durchgeführt wurden.

## Revendications

1. Procédé d'affichage d'un état de vigilance à l'intention d'un utilisateur, le procédé comprenant :
la détermination d'une classification de chronotype propre à l'utilisateur ;
l'alignement d'une courbe de vigilance associée à des niveaux de vigilance à différents moments sur la base de la classification du chronotype ;
l'affichage des niveaux de vigilance à partir de la courbe de vigilance à l'intention de l'utilisateur sur un écran (280) d'un dispositif portable (150) ;
l'affichage d'un test de vigilance sur le dispositif portable (150) ;
la réception d'un résultat du test de vigilance en provenance de l'utilisateur ;
la corrélation du résultat du test de vigilance avec un niveau de vigilance déterminé à partir de la courbe de vigilance ; et
la détermination d'un score de vigilance moyen sur la base du résultat d'une série de tests de vigilance effectués soit à un sommet, soit à un creux de la courbe de vigilance.

2. Procédé selon la revendication 1, comprenant en outre l'affichage de notifications pour des activités optimales à un moment prédéterminé sur la base de la classification du chronotype ; et/ou
dans lequel les notifications incluent des icônes correspondant à l'une des activités optimales affichées sur l'écran (280) ; et/ou
dans lequel le procédé comprend en outre l'affichage d'une interface de programme (420 ; 530) affichant un programme d'activités pour l'utilisateur qui inclut une entrée permettant à l'utilisateur d'insérer une activité optimale dans le programme d'activités.

3. Procédé selon l'une quelconque des revendications 1-2, comprenant en outre l'affichage d'une notification pour une durée de sommeil optimale sur la base de la classification du chronotype.

4. Procédé selon l'une quelconque des revendications 1-3, dans lequel le chronotype est déterminé sur la base des réponses de l'utilisateur à des questions d'un questionnaire.

5. Procédé selon l'une quelconque des revendications 1-4, dans lequel le chronotype est déterminé sur la base de données physiologiques mesurées chez l'utilisateur ; et/ou
dans lequel les données physiologiques sont déterminées par un capteur physiologique sur le dispositif portable (150) ; et/ou
dans lequel les données physiologiques sont analysées pour déterminer les heures de sommeil et d'éveil, ainsi que des niveaux d'activité tout au long de la journée afin de déterminer le chronotype ; et/ou
dans lequel des données opérationnelles d'un dispositif thérapeutique sont analysées pour déterminer les heures de sommeil et d'éveil, ainsi que des niveaux d'activité tout au long de la journée afin de déterminer le chronotype.

6. Procédé selon l'une quelconque des revendications 1-5, dans lequel la détermination est effectuée avec un modèle d'apprentissage automatique entraîné avec des données collectées auprès d'une population d'utilisateurs et avec le chronotype de chaque individu de la population d'utilisateurs.

7. Procédé selon l'une quelconque des revendications 1-6, comprenant en outre l'affichage de notifications invitant à manger ou à prendre des stimulants sur la base du chronotype ; et/ou
dans lequel le procédé comprend en outre l'affichage de notifications invitant à faire de l'exercice ou à travailler sur la base du chronotype ; et/ou
dans lequel le procédé comprend en outre la mesure de données relatives au sommeil chez l'utilisateur et l'affichage d'un niveau d'énergie prédit sur la base des données relatives au sommeil mesurées ; et/ou
dans lequel les données relatives au sommeil correspondent à un score de sommeil propre à l'utilisateur.

8. Procédé selon l'une quelconque des revendications 1-7, comprenant en outre l'affichage d'un indicateur visuel (416 ; 620) sur le dispositif portable (150) qui permet l'affichage d'un site Web sur un dispositif compatible avec le Web en scannant l'indicateur visuel ; et/ou
dans lequel l'indicateur visuel est un code QR (622) ; et/ou dans lequel le site Web est l'un parmi un site Web de coaching virtuel, un site Web d'information, ou un site Web de formation.

9. Procédé selon l'une quelconque des revendications 1-8, dans lequel le dispositif portable (150) inclut un émetteur-récepteur (266) qui permet une communication avec un dispositif mobile (150) ; et/ou dans lequel le dispositif portable (150) se porte au poignet.

10. Procédé selon l'une quelconque des revendications 1-9, dans lequel une représentation graphique (500) affichée sur l'écran (280) du dispositif portable (150) change en fonction de la période de temps et de la courbe de vigilance ; et/ou
dans lequel les niveaux d'alerte sont affichés en corrélation avec des couleurs prédéterminées sur l'écran (280).

11. Procédé selon l'une quelconque des revendications 1-10, comprenant en outre :
la collecte de données supplémentaires relatives au chronotype de l'utilisateur après la classification du chronotype ; et
l'ajustement de la courbe de vigilance sur la base des données collectées.

12. Procédé selon l'une quelconque des revendications 1-11, comprenant en outre l'alerte d'un utilisateur en cas de changements du niveau de vigilance sur la base de la courbe de vigilance.

13. Procédé selon l'une quelconque des revendications 1-12, comprenant en outre :
l'affichage d'un test de vigilance sur le dispositif portable ;
la réception d'un résultat du test de vigilance en provenance de l'utilisateur ; et
la corrélation du résultat du test de vigilance avec un niveau de vigilance déterminé à partir de la courbe de vigilance ; et/ou
dans lequel le test de vigilance est l'un parmi un test de mémoire à court terme, un test de réaction, ou un test de Stroop ; et/ou
dans lequel le procédé comprend en outre l'ajustement de la courbe de vigilance sur la base du résultat du test de vigilance.

14. Produit de programme informatique comprenant des instructions qui, lorsqu'elles sont exécutées par un ordinateur, amènent l'ordinateur à réaliser le procédé selon l'une quelconque des revendications 1 à 13.

15. Dispositif portable (150) permettant l'affichage de l'état de vigilance d'un utilisateur, le dispositif portable (150) comprenant :
un écran (280) ;
un dispositif de commande (260) couplé à l'écran (280), le dispositif de commande (260) étant apte à :
lire une entrée d'une classification de chronotype de l'utilisateur ;
aligner une courbe de vigilance relative à la vigilance de l'utilisateur par rapport à des périodes de temps sur la base de la classification du chronotype ;
afficher la courbe de vigilance sur l'écran (280) ;
afficher un test de vigilance sur l'écran (280) ;
recevoir de l'utilisateur un résultat du test de vigilance ;
corréler le résultat du test de vigilance avec un niveau de vigilance déterminé à partir de la courbe de vigilance ; et
déterminer un score de vigilance moyen sur la base du résultat d'une série de tests de vigilance effectués soit à un sommet, soit à un creux de la courbe de vigilance.
